# EUROPÄISCHE PATENTSCHRIFT

(11) **EP 1 218 320 B1**
(45) Veröffentlichungstag und Bekanntmachung des Hinweises auf die Patenterteilung: **26.05.2004**
(21) Anmeldenummer: 00969378.9
(22) Anmeldetag: 02.10.2000
(51) Int. Cl.: C07B 63/04, C09K 15/30, C07C 51/50

(54) **BEHANDLUNG VON GEMISCHEN, DIE WENIGSTENS EINE ETHYLENISCH UNGESÄTTIGTE VERBINDUNG ENTHALTEN**
TREATMENT OF MIXTURES THAT CONTAIN AT LEAST ONE COMPOUND WITH AT LEAST ONE ETHYLENICALLY UNSATURATED GROUP
PROCEDE POUR TRAITER DES MELANGES CONTENANT AU MOINS UN COMPOSE COMPORTANT AU MOINS UN GROUPE ETHYLENIQUEMENT INSATURE

(30) Priorität: 05.10.1999 DE 19947868
(43) Veröffentlichungstag der Anmeldung: 03.07.2002
(73) Patentinhaber: BASF AKTIENGESELLSCHAFT, 67056 Ludwigshafen (DE)
(72) Erfinder: MÜLLER-ENGEL, Klaus, Joachim, 76297 Stutensee (DE); NESTLER, Gerhard, 67061 Ludwigshafen (DE); SCHRÖDER, Jürgen, 67071 Ludwigshafen (DE)
(86) Internationale Anmeldenummer: PCT/EP2000/009630
(87) Internationale Veröffentlichungsnummer: WO 2001/025173

(56) Entgegenhaltungen:
- EP-A- 0 178 168
- EP-A- 0 765 856
- US-A- 3 271 296
- US-A- 5 496 875

## Beschreibung

Vorliegende Erfindung betrifft ein Verfahren der chemischen und/oder physikalischen Behandlung von Gemischen, die wenigstens eine chemische Verbindung mit wenigstens einer ethylenisch ungesättigten Gruppe enthalten.

Chemische Verbindungen, die wenigstens eine ethylenisch ungesättigte Gruppe enthalten (Monomere) sind allgemein bekannt und bilden wichtige Ausgangsverbindungen zur Herstellung von Polymerisaten (z.B. durch radikalische Polymerisation), die u.a. als Klebstoffe oder als Bindemittel Verwendung finden.

Im Rahmen der Herstellung von Monomeren wie z. B. (Meth)acrylsäure ("Meth)acryl-" wird in dieser Schrift verkürzend für "Acryl- oder Methacryl-" verwendet), Ester der (Meth)acrylsäure, Nitrile der (Meth)acrylsäure oder Styrol ist es in an sich bekannter Weise immer wieder erforderlich, Gemische, die wenigstens ein Monomer enthalten, chemischen und/oder physikalischen Behandlungen zu unterwerfen.

Beispielhaft genannt sei die Veresterung von (Meth)acrylsäure mit ein- oder mehrwertigen Alkanolen (vgl. z. B. EP-A 463 434) oder die rektifikative Behandlung von (Meth)acrylsäure enthaltenden Gemischen (vgl. z. B. DE-A 19 810 962 oder EP-A 648 732).

Nachteilig an diesen bekannten Verfahren der chemischen und/oder physikalischen Behandlung von Gemischen, die wenigstens eine chemische Verbindung mit wenigstens einer ethylenisch ungesättigten Gruppe enthalten, ist, daß Monomere zur unerwünschten radikalischen Polymerisation neigen, weshalb die Verfahren der chemischen und/oder physikalischen Behandlung von Gemischen, die wenigstens eine chemische Verbindung mit wenigstens einer ethylenisch ungesättigten Gruppe enthalten, üblicherweise im Beisein von radikalischen Polymerisationsinhibitoren durchgeführt werden. Als solche radikalischen Polymerisationsinhibitoren sind z. B. Nitroxyl-Radikale (Verbindungen die wenigstens eine >N-O●-Gruppe aufweisen) bekannt (vgl. z. B. WO 9 921 893, US-A 4 670 131 und EP-A 765 856).

Aber auch bei Mitverwendung von radikalischen Polymerisationsinhibitoren läßt sich eine unerwünschte radikalische Polymerisation von Monomeren häufig nicht ausschließen, weshalb bei Verfahren der chemischen und/oder physikalischen Behandlung von Gemischen, die wenigstens eine chemische Verbindung mit wenigstens einer ehtylenisch ungesättigten Gruppe enthalten, neben Polymerisationsinhibitoren häufig noch Substanzen zugesetzt werden, die in unerwünschter Weise gebildetes Polymerisat in Schwebe halten, d. h., eine Ausbildung von Polymerisatablagerungen auf z. B. Behälterwänden, Kolonnenböden oder Verdampferoberflächen verhindern sollen. Derartige Substanzen werden als Antifoulingmittel bezeichnet (vgl. z. B. US-A 3 271 296).

Die Aufgabe der vorliegenden Erfindung bestand vor diesem Hintergrund darin, Verfahren der chemischen und/oder physikalischen Behandlung von Gemischen, die wenigstens eine chemische Verbindung mit wenigstens einer ethylenisch ungesättigten Gruppe enthalten, zur Verfügung zu stellen, die im Beisein von Substanzen durchgeführt werden, die einerseits hervorragende radikalische Polymerisationsinhibitoren und andererseits hervorragende Antifoulingmittel bilden.

Demgemäß wurde ein Verfahren der chemischen und/oder physikalischen Behandlung von Gemischen, die wenigstens eine chemische Verbindung mit wenigstens einer ethylenisch ungesättigten Gruppe enthalten, gefunden, das dadurch gekennzeichnet ist, daß es im Beisein von wenigstens einer Verbindung der allgemeinen Formeln (I), (II) 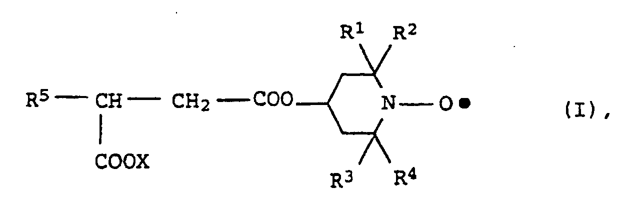 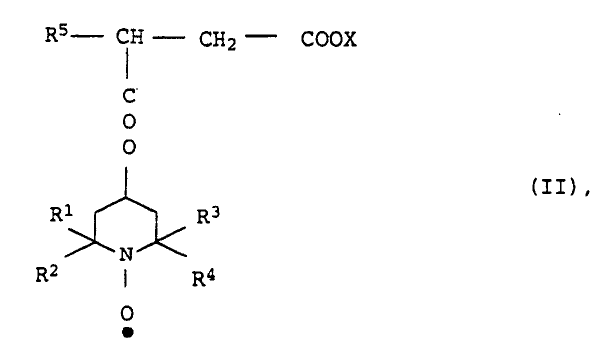
- mit X =: H, ein Alkalimetall und/oder Ammonium
- R¹, R², R³ und R⁴ =: unabhängig voneinander C₁- bis C₄-Alkyl
und
- R⁵ =: C₈- bis C₃₀-Alkyl,
durchgeführt wird.

Verbindungen der allgemeinen Formeln (I), (II) sind z. B aus der US-A 5 496 875 bekannt und werden dort als Zwischenprodukte zur Herstellung von Licht- und Hitzestabilisatoren von Polymerisaten empfohlen.

Als Alkalimetall X kommen erfindungsgemäß insbesondere Na und K in Betracht. Die Reste R¹, R², R³ und R⁴ können unabhängig voneinander Methyl, Ethyl, n-Propyl, iso-Propyl, n-Butyl, iso-Butyl oder tert.-Butyl bedeuten. Erfindungsgemäß geeignet sind demnach auch Verbindungen (I), (II), in denen alle Reste R¹, R², R³ und R⁴ Methyl oder in denen alle Reste R¹, R², R³ und R⁴ Ethyl sind. R⁵ kann unter anderem C₁₅- bis C₂₅-Alykl oder C₁₇- bis C₂₂-Alykl bedeuten.

Die Herstellung von Verbindungen (I), (II) kann ebenfalls der US-A 5 496 875 entnommen werden.

Zur Herstellung der Verbindungen 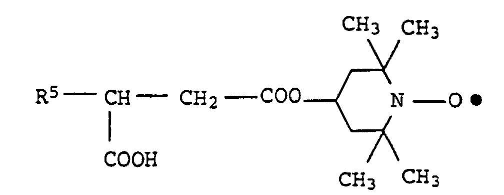 bzw. 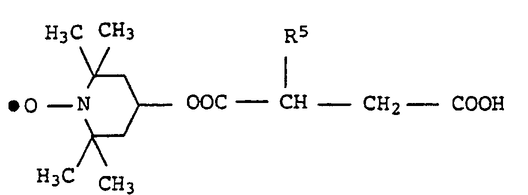 können z. B. die entsprechenden Alkylbernsteinsäureanhydride bei Temperaturen von 60 bis 120°C mit 4-Hydroxy-2,2,6,6-tetramethylpiperidin-N-oxyl (HTEMPO) umgesetzt werden. In der Regel wird man dazu das Molverhältnis von Anhydrid: N-oxyl zu 0,8 bis 1,5 : 1 wählen. Vorzugsweise führt man die Umsetzung in Abwesenheit eines Lösungsmittel durch. Als solche geeigneten Lösungsmittel kommen beispielsweise in Betracht aromatische und/oder aliphatische Kohlenwasserstoffe wie Toluol, Xylol und Cyclohexan, aber auch Diphenylether, Dialkylphthalate, Dialkylacetamide und N-Alkylpyrrolidone. Die Reaktionszeit beträgt in der Regel 0,1 - 5 Stunden. Mit Vorteil erfolgt die Synthese unter wasserfreien Bedingungen.

Als Alkylbernsteinsäureanhydride können z. B. Verbindungen wie Tetrapropenylbernsteinsäureanhydrid (z. B. GP 103 der Fa. CONDEA), n-Alkenylbernsteinsäureanhydrid mit einer zahlenmittleren Molmasse (Mₙ) von ca. 520 (z. B. GP 104 der Fa. CONDEA), Polyisobutenylbernsteinsäureanhydrid mit Mₙ ca. 850 (z. B. GP 105 der Fa. CONDEA) oder Mₙ ca. 1400 (z. B. GP 106 der Fa. CONDEA) verwendet werden.

Mögliche, wenigstens eine ethylenisch ungesättigte Gruppe aufweisende Verbindungen können im Rahmen des erfindungsgemäßen Verfahrens z. B. Styrol, Butadien, Ethylen, Vinylether, Vinylester, Acrylsäure, Methacrylsäure, Alkylester (insbesondere C₁- bis C₈-Alkyl) der Acrylsäure und Methacrylsäure, Methacrylnitril, Acrylnitril oder N-Vinylpyrrolidon sein.

Bezogen auf die Menge der im erfindungsgemäß zu behandelnden Gemisch enthaltenen Monomeren werden die erfindungsgemäß mitzuverwendenden Verbindungen I, II in der Regel in Mengen von 50 bis 1000 gew.ppm eingesetzt. Selbstverständlich kann die Einsatzmenge in entsprechender Weise bezogen aber auch bis zu 2000 oder bis zu 3000 gew.ppm und mehr betragen. Selbstredend kann die Einsatzmenge in geeigneten Fällen aber auch weniger als 50 gew.ppm betragen.

Vorzugsweise wird man die erfindungsgemäß zu verwendenden Verbindungen (I), (II) so wählen, daß sie in der benötigten Einsatzmenge im erfindungsgemäß zu behandelnden Gemisch löslich sind.

In der Regel wird man erfindungsgemäß Gemische aus Verbindungen I und II verwenden.

Selbstverständlich können die Verbindungen I und II in den erfindungsgemäßen Verfahren auch im Gemisch mit anderen, bekannten, Polymerisationsinhibitoren und/oder Antifoulingmitteln zum Einsatz kommen. Als solche kommen u.a. in Betracht: Luft, Hydrochinon, Hydrochinonmonoethylether (MEHQ), Paranitrosophenol, Paramethoxyphenol, Phenothiazin (PTZ), Phenylendiamine, 4-Hydroyxy-2,2,6-6-tetramethyl-piperidin-N-oxyl (HTEMPO), organische Sulfonsäuren (z. B. die in der EP-A 648 732 veröffentlichten), Tenside (z. B. die in der DE-A 19810962 erwähnten) sowie alle in der WO 9921893 genannten Polymerisationsinhibitoren.

Günstige Kombinationen sind z.B.
a) Verbindungen I, II / PTZ;
b) Verbindungen I, II / PTZ / MEHQ;
c) Verbindungen I, II / PTZ / MEHQ / HTEMPO;
d) Verbindungen I, II / MEHQ / HTEMPO;
e) Verbindungen I, II / MEHQ;
f) Verbindungen I, II / MEHQ / HTEMPO.

Bei der erfindungsgemäßen chemischen Behandlung kann es sich z.B. um eine an sich bekannte chemische Umsetzung eines Monomeren unter Erhalt der wenigstens einen ehtylenisch ungesättigten Gruppe handeln. Als Beispiel wurde bereits die Veresterung von z.B. (Meth)acrylsäure mit Alkanolen genannt. Bei der erfindungsgemäßen physikalischen Behandlung kann es sich zum Beispiel um Extraktionsverfahren, Destillationsverfahren, Rektifikationsverfahren, Absorptionsverfahren oder Kristallisationsverfahren handeln.

Dabei kann es sich bei den erfindungsgemäß zu behandelnden Gemischen z. B. um reine Monomerengemische aber auch um Gemische aus Monomeren und von Monomeren verschiedenen Substanzen handeln. In der Regel beträgt der Gewichtsanteil der Monomeren an den erfindungsgemäß zu behandelnden Gemischen wenigstens 5 Gew.-%, oder wenigstens 10 Gew.-%, oder wenigstens 15 Gew.-% bzw. 25 Gew.-% oder 40 Gew.-%.

Insbesondere kann das erfindungsgemäß zu behandelnde Gemisch zu ≥ 95 Gew.-% aus (Meth)acrylsäure bestehen.

Unter anderem eignet sich das erfindungsgemäße Verfahren zur rektifikativen Abtrennung von (Meth)acrylsäure aus einem (Meth)acrylsäure und eine höher als (Meth)acrylsäure siedende organische Flüssigkeit als Hauptbestandteile enthaltenden Gemisch, wie es in der DE-A 19810962 beschrieben ist. Dabei können die erfindungsgemäß zu verwendenden Verbindungen I, II an allen Stellen der rektifikativen Abtrennung zugeführt werden, an denen die WO 9921893 einen Tensidzusatz empfiehlt. Bei Bedarf können sie gemeinsam mit Tensiden angewendet werden. Häufig wird man die Verbindungen I, II in (Meth)acrylsäure gelöst zusetzen. Darüber hinaus eignet sich die erfindungsgemäße Verfahrensweise auch bei den Verfahren der destillativen Reinigung von Roh-(Meth)acrylsäure, wie sie in der EP-A 648 732 beschrieben sind. Dabei können die erfindungsgemäß einzusetzenden Verbindungen I, II alternativ oder gemeinsam mit den in der EP-A 648 732 verwendeten Polymerisationsinhibitoren und Sulfonsäuren verwendet werden.

### Beispiele

a) Jeweils 1 g einer mit 300 gew.ppm Phenothiazin stabilisierten Roh-Acrylsäure, die durch katalytische Gasphasenoxidation von Acrolein gemäß Beispiel B1 der DE-A 4 302 991 und anschließende Aufarbeitung des Reaktionsgasgemisches gemäß Beispiel B1 der DE-A 2 136 396 erhalten worden war, wurde luftgesättigt mit verschiedenen Mengen verschiedener Polymerisationsinhibitoren und/oder Antifoulingmittel in einem Probenröhrchen vermischt.
Anschließend wurden jeweils 5 mg Azobisisobutyronitril (radikalischer Polymerisationsinitiator) zugegeben und die Proben offen im Wasserbad bei 60°C temperiert.
Dann wurde die Zeitdauer ermittelt, die verging, bis die Probe zu polymerisieren begann (Detektor: die frei werdende Polymerisationswärme). Die in Abhängigkeit vom zugesetzten Polymerisationsinhibitor und/oder Antifoulingmittel erhaltenen Zeiten zeigt die nachfolgende Tabelle 1. Die Mengenangaben beziehen sich auf die Gesamtmenge des Gemischs. Bei Verzicht auf jeglichen zusätzlichen Inhibitor- und/oder Antifoulingzusatz (d.h., bei alleiniger Verwendung der Ausgangs-Roh-Acrylsäure) betrug die Zeitdauer 19 min.

**Tabelle 1**

| Zugesetztes Mittel | Zeit (min) |
|---|---|
| 300 gew.ppm GP 103 (von CONDEA) | 19 |
| 600 gew.ppm GP 104 (von CONDEA) | 20 |
| 300 gew.ppm HTEMPO | 25 |
| 300 gew.ppm GP 103 (von CONDEA) und 300 gew.ppm HTEMPO | 24 |
| 600 gew.ppm des Umsetzungsproduktes von GP 103 (von CONDEA) mit HTEMPO | 24 |

Bemerkenswerterweise wird die die radikalische Polymerisation inhibierende Wirkung von HTEMPO durch die chemische Anbindung von GP 103 nicht beeinträchtigt.
b) In eine kontinuierlich zu betreibende Rektifikationseinheit aus Glas, deren Verdampfer ein Konvektionsumlaufverdampfer war, der mittels einer metallischen, elektrisch beheizbaren Kerze beheizt wurde, wurden über den Verdampfer kontinuierlich 137 g/h einer Roh-Acrylsäure zugeführt, die durch katalytische Gasphasenoxidation von Acrolein gemäß Beispiel B1 der DE-A 4 302 991 und anschließende Aufarbeitung der Reaktionsgase gemäß Beispiel B1 der DE-A 2 136 396 erhalten worden war und der vor der Zufuhr in den Verdampfer 1100 gew.ppm Aminoguanidinhydrogencarbonat (als Aldehydfänger) und die zu testenden Polymerisationsinhibitoren und/oder Anitfoulingmittel (vgl. Tabelle 2) zugesetzt wurden. Die Temperatur im Kreisdampfer betrug 78°C, der Druck am Kopf der Kolonne lag bei 100 mbar. Die Kolonne war 1,5 m lang und mit Raschigringen (5 mm, Glas) gefüllt.
Das über die Kolonne ausgeschleuste Gemisch aus in der Roh-Acrylsäure enthaltenen Leichtsiedern wie Essigsäure und Wasser sowie geringen Mengen an Acrylsäure wurde kondensiert. 25 g/h des Kondensats wurden ausgeschleust und der Rest als Rücklauf am Kolonnenkopf wieder zugeführt. Zur Stabilisierung der Kolonne wurde an deren Kopf eine Lösung von 5000 gew.ppm Phenothiazin in reiner Acrylsäure aufgegeben (20 ml/h). Das von den Leichtsiedern weitgehend befreite Sumpfprodukt wurde standgeregelt aus dem Verdampfer entfernt. Während der Rektifikation trat auf der Heizkerze Belagsbildung auf, deren Menge in Abhängigkeit vom zu testenden Polymerisationsinhibitor und/oder Antifoulingmittel nach einer Betriebsdauer von jeweils 40 h ausgewogen wurde. Die erhaltenen Ergebnisse zeigt die Tabelle 2.

**Tabelle 2**

| Zugesetztes Mittel | Belagsbildung |
|---|---|
| 100 gew.ppm HTEMPO | 7,7 g |
| 100 gew.ppm HTEMPO und 100 gew.ppm GP 103 (von CONDEA) | 4,2 g |
| 200 gew.ppm des Umsetzungsproduktes von GP 103 (von CONDEA) mit HTEMPO | 1,4 g |
| 400 gew.ppm des Umsetzungsproduktes von GP 104 (von CONDEA) mit HTEMPO | 1,1 g |
| 600 gew.ppm des Umsetzungsproduktes von GP 105 (von CONDEA) mit HTEMPO | 1,2 g |

Bemerkenswerterweise wirken die erfindungsgemäß zu verwendenden Verbindungen I, II nicht nur in hervorragender Weise als Polymerisationsinhibitoren, sondern auch als ausgezeichnete Antifoulingmittel.

## Patentansprüche

1. Verfahren der chemischen und/oder physikalischen Behandlung von Gemischen, die wenigstens eine chemische Verbindung mit wenigstens einer ethylenisch ungesättigten Gruppe enthalten, **dadurch gekennzeichnet, daß** es im Beisein von wenigstens einer Verbindung der allgemeinen Formeln (I), (II) 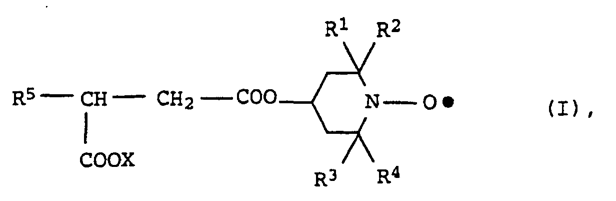 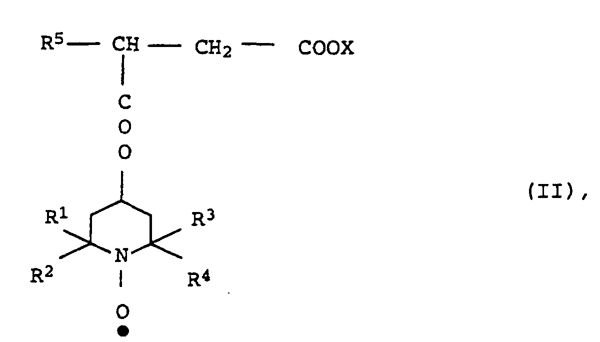 mit
X = H, ein Alkalimetall und/oder Ammonium,
R¹, R², R³ und R⁴ = unabhängig voneinander C₁- bis C₄-Alkyl
und
R⁵ = C₈- bis C₃₀-Alkyl,
durchgeführt wird.

2. Verfahren nach Anspruch 1, **dadurch gekennzeichnet, daß** die wenigstens eine chemische Verbindung mit wenigstens einer ethylenisch ungesättigten Gruppe Acrylsäure, Methacrylsäure, Acrylnitril, Methacrylnitril, Styrol, ein Ester der Acrylsäure und/oder ein Ester der Methacrylsäure ist.

3. Verfahren nach Anspruch 1 oder 2, **dadurch gekennzeichnet, daß** das zu behandelnde Gemisch ein (Meth)acrylsäure und eine höher als (Meth)acrylsäure siedende organische Flüssigkeit als Bestandteile enthaltendes Gemisch ist.

4. Verfahren nach einem der Ansprüche 1 bis 3, **dadurch gekennzeichnet, daß** das zu behandelnde Gemisch zu ≥ 95 Gew.-% aus (Meth) acrylsäure besteht.

5. Verfahren nach einem der Ansprüche 1 bis 4, **dadurch gekennzeichnet, daß** es sich um ein Rektifikations-, Extraktionsoder Absorptionsverfahren handelt.

6. Verfahren nach einem der Ansprüche 1 bis 5, **dadurch gekennzeichnet, daß** R¹, R², R³ und R⁴ entweder alle Methyl und/oder alle Ethyl sind.

7. Verfahren nach einem der Ansprüche 1 bis 6, **dadurch gekennzeichnet, daß** X = H ist.

8. Verfahren nach einem der Ansprüche 1 bis 7, **dadurch gekennzeichnet, daß** es bei einer Temperatur von 100 bis 200°C durchgeführt wird.

9. Verfahren nach einem der Ansprüche 1 bis 8, **dadurch gekennzeichnet, daß** es bei einem Druck ≤ 100 mbar durchgeführt wird.

10. Gemisch, das wenigstens eine chemische Verbindung mit wenigstens einer ethylenisch ungesättigten Gruppe und wenigstens eine Verbindung der allgemeinen Formeln (I), (II) 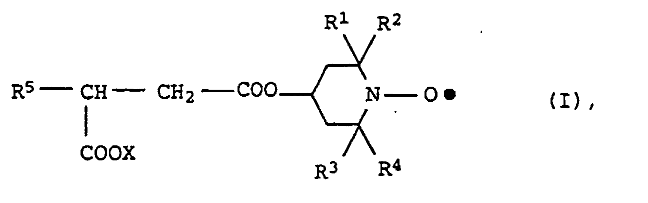 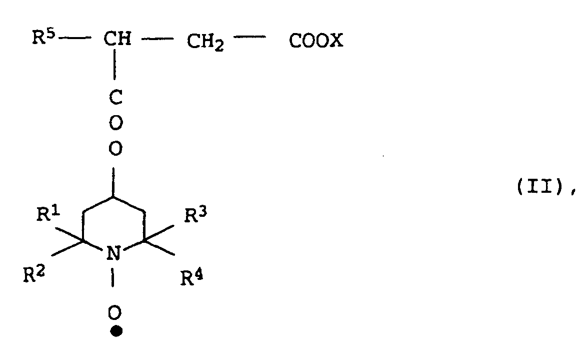 mit
X = H, ein Alkalimetall und/oder Ammonium,
R¹, R², R³ und R⁴ = unabhängig voneinander C₁- bis C₄-Alkyl
und R⁵ = C₈- bis C₃₀-Alkyl,
enthält.

11. Gemisch nach Anspruch 10, bei dem die wenigstens eine chemische Verbindung mit wenigstens einer ethylenisch ungesättigten Gruppe ausgewählt ist aus der Gruppe umfassend Acrylsäure, Methacrylsäure, Acrylnitril, Methacrylnitril, Styrol, Ester der Acrylsäure und Ester der Methacrylsäure.

12. Gemisch nach Anspruch 10 oder 11, wobei R¹, R², R³ und R⁴ entweder alle Methyl und/oder alle Ethyl sind.

13. Verfahren der chemischen und/oder physikalischen Behandlung von Gemischen, die wenigstens eine chemische Verbindung mit wenigstens einer ethylenisch ungesättigten Gruppe enthalten, **dadurch gekennzeichnet, daß** es im Beisein von wenigstens einer Verbindung durchgeführt wird, die durch Umsetzung eines n-Alkenylbernsteinsäureanhydrids mit einer zahlenmittleren Molmasse (Mₙ) von ca. 520, oder eines Polyisobutenylbernsteinsäureanhydrid mit Mₙ ca. 850 oder ca. 1400 mit 4-Hydroxy-2,2,6,6-tetramethyl-piperidin-N-oxyl erhältlich ist.

## Claims

1. A process for the chemical and/or physical treatment of a mixture which contains at least one chemical compound having at least one ethylenically unsaturated group, which is carried out in the presence of at least one compound of the formulae (I) and (II) 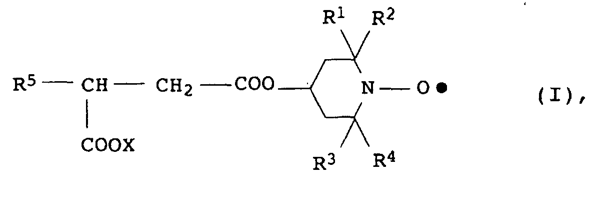 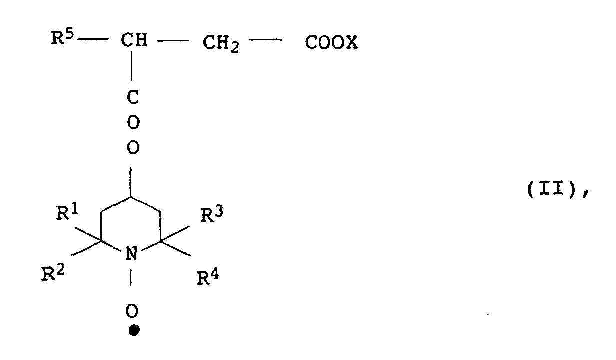 where
X is H, an alkali metal and/or ammonium,
R¹, R², R³ and R⁴, independently of one another, are each C₁- to C₄-alkyl and
R⁵ is C₈- to C₃₀-alkyl.

2. A process as claimed in claim 1, wherein the at least one chemical compound having at least one ethylenically unsaturated group is acrylic acid, methacrylic acid, acrylonitrile, methacrylonitrile, styrene, an ester of acrylic acid and/or an ester of methacrylic acid.

3. A process as claimed in claim 1 or 2, wherein the mixture to be treated is a mixture containing, as components, (meth)acrylic acid and an organic liquid having a higher boiling point than (meth)acrylic acid.

4. A process as claimed in any of claims 1 to 3, wherein the mixture to be treated comprises ≥ 95% by weight of (meth)acrylic acid.

5. A process as claimed in any of claims 1 to 4, which is a rectification, extraction or absorption process.

6. A process as claimed in any of claims 1 to 5, wherein R¹, R², R³ and R⁴ are either all methyl and/or all ethyl.

7. A process as claimed in any of claims 1 to 6, wherein X is H

8. A process as claimed in any of claims 1 to 7, which is carried out at from 100 to 200°C.

9. A process as claimed in any of claims 1 to 8, which is carried out at ≤ 100 mbar.

10. A mixture which contains at least one chemical compound having at least one ethylenically unsaturated group and at least one compound of the formulae (I) and (II) 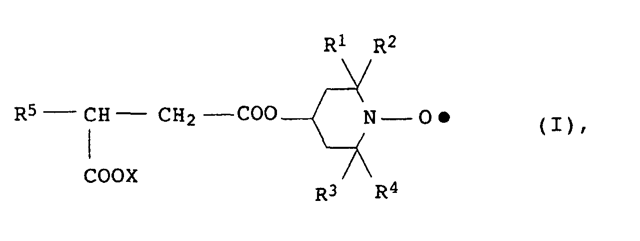 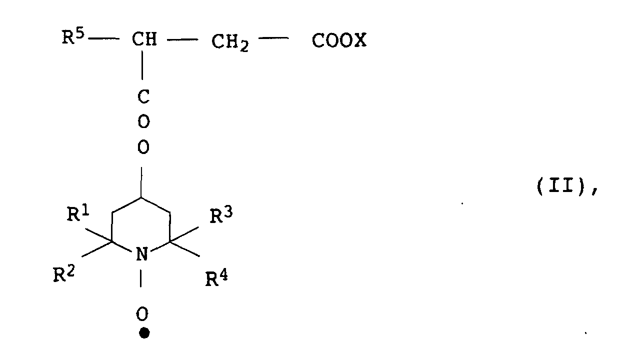 where
X is H, an alkali metal and/or ammonium,
R¹, R², R³ and R⁴ , independently of one another, are each C₁- to C₄-alkyl and
R⁵ is C₈- to C₃₀-alkyl.

11. A mixture as claimed in claim 10, in which the at least one chemical compound having at least one ethylenically unsaturated group is selected from the group consisting of acrylic acid, methacrylic acid, acrylonitrile, methacrylonitrile, styrene, esters of acrylic acid and esters of methacrylic acid.

12. A mixture as claimed in claim 10 or 11, wherein R¹, R², R³ and R⁴ are either all methyl and/or all ethyl.

13. A process for the chemical and/or physical treatment of a mixture which contains at least one chemical compound having at least one ethylenically unsaturated group, which is carried out in the presence of at least one compound which is obtainable by reacting an n-alkenylsuccinic anhydride having a number average molar mass (Mₙ) of about 520 or a polyisobutenylsuccinic anhydride where Mₙ is about 850 or about 1400 with 4-hydroxy-2,2,6,6-tetramethylpiperidin-N-oxyl.

## Revendications

1. Procédé de traitement chimique et/ou physique de mélanges, qui contiennent au moins un composé chimique comportant au moins un groupe éthyléniquement insaturé, **caractérisé en ce qu'**il est effectué en présence d'au moins un composé des formules générales (I), (II) : 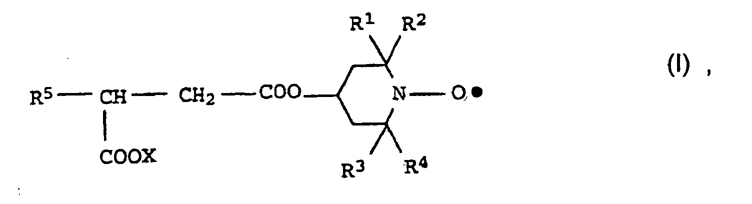 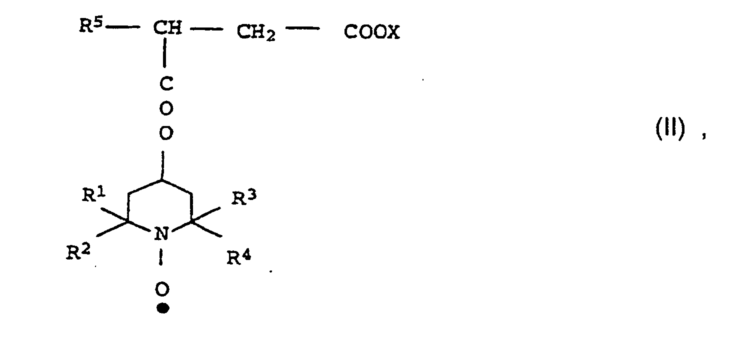 dans lesquelles
X = H, un métal alcalin et/ou de l'ammonium,
R¹, R², R³ et R⁴ = indépendamment l'un de l'autre de l'alkyle en C₁-C₄,
et R⁵ = de l'alkyle en C₈-C₃₀.

2. Procédé suivant la revendication 1, **caractérisé en ce que** ledit au moins un composé chimique comportant au moins un groupe éthyléniquement insaturé est de l'acide acrylique, de l'acide méthacrylique, de l'acrylonitrile, du méthacrylonitrile, du styrène, un ester de l'acide acrylique et/ou un ester de l'acide méthacrylique.

3. Procédé suivant l'une des revendications 1 et 2, **caractérisé en ce que** le mélange à traiter est un mélange contenant, comme composants, de l'acide (méth)acrylique et un liquide organique à point d'ébullition supérieur à l'acide (méth)acrylique.

4. Procédé suivant l'une des revendications 1 à 3, **caractérisé en ce que** le mélange à traiter est constitué d'acide (méth)acrylique pour ≥ 95% en poids.

5. Procédé suivant l'une des revendications 1 à 4, **caractérisé en ce qu'**il s'agit d'un procédé de rectification, d'extraction ou d'absorption.

6. Procédé suivant l'une des revendications 1 à 5, **caractérisé en ce que** R¹, R², R³ et R⁴ représentent tous du méthyle et/ou tous de l'éthyle.

7. Procédé suivant l'une des revendications 1 à 6, **caractérisé en ce que** X représente H.

8. Procédé suivant l'une des revendications 1 à 7, **caractérisé en ce qu'**il est effectué à une température de 100 à 200°C.

9. Procédé suivant l'une des revendications 1 à 8, **caractérisé en ce qu'**il est effectué à une pression ≤ 100 mbars.

10. Mélange, qui contient au moins un composé chimique comportant au moins un groupe éthyléniquement insaturé et au moins un composé des formules générales (I), (II) : 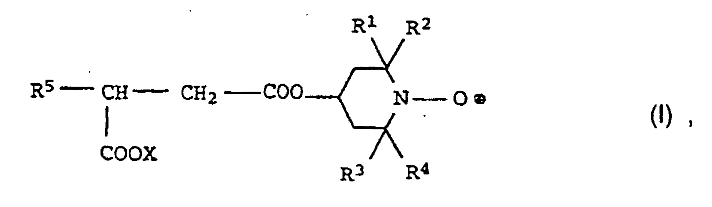 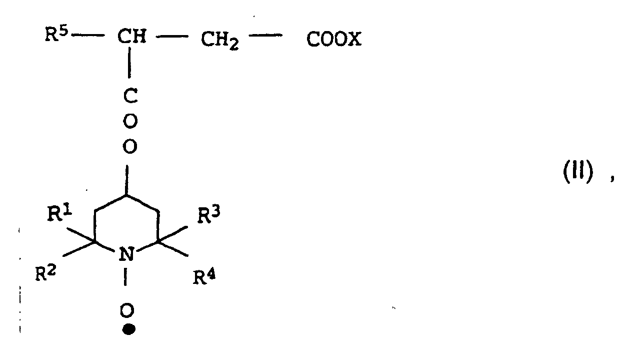 dans lesquelles
X = H, un métal alcalin et/ou de l'ammonium,
R¹, R², R³ et R⁴ = indépendamment l'un de l'autre de l'alkyle en C₁-C₄,
et R⁵ = de l'alkyle en C₈-C₃₀.

11. Mélange suivant la revendication 10, dans lequel ledit au moins un composé chimique comportant au moins un groupe éthyléniquement insaturé est choisi parmi le groupe comprenant de l'acide acrylique, de l'acide méthacrylique, de l'acrylonitrile, du méthacrylonitrile, du styrène, de l'ester d'acide acrylique et de l'ester d'acide méthacrylique.

12. Mélange suivant l'une des revendications 10 et 11, dans lequel R¹, R², R³ et R⁴ représentent tous du méthyle et/ou tous de l'éthyle.

13. Procédé de traitement chimique et/ou physique de mélanges, qui contiennent au moins un composé chimique comportant au moins un groupe éthyléniquement insaturé, **caractérisé en ce qu'**il est effectué en présence d'au moins un composé qui peut être obtenu par réaction d'un anhydride n-alcénylsuccinique présentant une masse molaire moyenne numérique (Mₙ) d'environ 520 ou d'un anhydride polyisobuténylsuccinique présentant un Mₙ d'environ 850 ou d'environ 1.400 avec du 4-hydroxy-2,2,6,6-tétraméthyl-pipéridine-N-oxyle.
